(19) Europäisches Patentamt
European Patent Office
Office européen des brevets

(11) Publication number: **0 248 656 B1**

(12) **EUROPEAN PATENT SPECIFICATION**

(45) Date of publication of patent specification: **31.03.93**

(51) Int. Cl.[5]: **C12N 5/00**

(21) Application number: **87304919.1**

(22) Date of filing: **03.06.87**

The file contains technical information submitted after the application was filed and not included in this specification

(54) **Composition for cell cultivation and use thereof.**

(30) Priority: **04.06.86 JP 128112/86**
**27.09.86 JP 227190/86**
**26.11.86 JP 279773/86**
**26.11.86 JP 279774/86**

(43) Date of publication of application:
**09.12.87 Bulletin 87/50**

(45) Publication of the grant of the patent:
**31.03.93 Bulletin 93/13**

(84) Designated Contracting States:
**CH DE FR GB LI NL**

(56) References cited:
**WO-A-88/00967**
**DE-A- 1 226 744**
**FR-A- 2 527 448**
**FR-A- 2 559 499**

**ANALYTICAL BIOCHEMISTRY, vol. 130, 1983, pages 445-453, Academic Press INC., T. KAWAMOTO et al.: "Development of a serum-free medium for growth of NS-1 mouse myeloma cells and its application to the isolation of NS-1 hybridomas"**

(73) Proprietor: **Director-General of the Agency of Industrial Science and Technology**
**Ministry of International Trade & Industry 3-1 Kasumigaseki 1-chome**
**Chiyoda-ku Tokyo 100(JP)**

(72) Inventor: **Kitano, Kazuaki**
**15-4, Akasakadai 4-cho**
**Sakai Osaka 590-01(JP)**
Inventor: **Shintani, Yasushi**
**5-49, Hananzonohonmachi 1-cho**
**Higashiosaka**
**Osaka 578(JP)**

(74) Representative: **Lewin, John Harvey et al**
**Elkington and Fife Prospect House 8 Pembroke Road**
**Sevenoaks, Kent TN13 1XR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

### INDUSTRIAL FIELD TO BE APPLIED

This invention relates to a composition for cultivating animal cells, a process for cultivating animal cells and a process for preparing biologically active substances.

### PRIOR ART

Technologies of efficiently culturing animal cells in large scales have been investigated from various points of view for the research and production of new useful biologically active substances and in addition as essential technology for the production of biologically active substances using cells which have been subjected to genetic manipulation technique. For the culture of animal cells in prior art, there have been generally employed culture mediums to which serum had been added in an amount of about 10%, among which recommended a fetal calf serum (FCS) containing medium.

### PROBLEMS INTENDED TO SOLVE BY THE INVENTION

However, serum is very expensive and usually has an illdefined lot variation, the culture of cells in a large amount using such a medium arises many problems. Furthermore, serum contains various kinds of heteroproteins, and thus there happen many disadvantages on the recovery and purification of useful substances produced from the medium containing serum. For the purpose of dissolving such problems, a variety of media containing no serum (serum-free media) were developed, but they generally have low applicability and are not always satisfactory in the growth ability of cells and the productivity of biologically active substances as compared with serum-containing media. In serum-free media, a variety of cell growth factors, hormones and the like are added as substituents of serum, but many of these substances are expensive and thus the serum-free media often become more expensive than serum-containing media.

In any event, conventional media known to the art were not always satisfactory for effectively obtaining a useful substance in a large amount by cell culture.

Therefore, it is desired to develop a culture medium for growing animal cells which medium can be supplied inexpensively and in a large amount and does not contain as far as possible any protein derived from serum or the like of which property remains ambiguous. As the culture medium, a medium which is serum-free and has a wide applicability and a high cell cultivation capacity is ideal. But when the culture medium is a serum containing medium, it will be possible to improve extensively the problems of medium economy and the content of contamination in culture medium if the amount of serum used can be reduced extensively.

### MEANS FOR SOLVING PROBLEMS

Based on the aforementioned considerations, the present inventors have conducted a variety of researches. As a result thereof, they have found that when an animal cell or the biologically active substance producing animal cell is cultured in a medium which is a composition for growing animal cells and contains one or more of polyethylene glycol, polyvinyl alcohol and polypropylene glycol and additionally a low density lipoprotein, the animal cell is grown remarkably and thus the amount of a biologically active substance to be produced is increased. As the result of further research based on these findings, they have achieved this invention.

That is to say, this invention provides (1) a serum-free composition for growing animal cells which contains one or more of polyethylene glycol in a concentration of 10% (w/v) or less, polyvinyl alcohol and polypropylene glycol and in addition, a low density lipoprotein (2) a process for growing animal cells, comprising growing animal cells in a serum-free medium containing one or more of polyethylene glycol in a concentration of 10% (w/v) or less, polyvinyl alcohol and polypropylene glycol and in addition, a low density lipoprotein and (3) a process for producing a biologically active substance, comprising growing a biologically active substance-producing animal cells in a serum-free medium containing one or more of polyethylene glycol in a concentration of 10% (w/v) or less, polyvinyl glycol and polypropylene glycol and in addition, a low density lipoprotein, producing and accumulating said substance in the culture and collecting the substance.

In this specification, polyethylene glycol, polyvinyl alcohol, polypropylene glycol and low density lipoprotein are referred to as PEG, PVA, PPG and LDL, respectively.

BRIEF DESCRIPTION OF THE DRAWINGS

Fig. 1 illustrates the effect of adding polyethylene glycol obtained in Example 1;
Fig. 2 illustrates the effect of adding polyvinyl alcohol obtained in Example 2;
Fig. 3 illustrates the effect of adding polypropylene glycol obtained in Example 2;
Fig. 4 illustrates the effect of adding various concentrations of human LDL obtained in Example 4;
Fig. 5 illustrates the effect of adding various concentrations of swine LDL obtained in Example 5; and
Fig. 6 illustrates the result of serial transfer of the cell obtained in Example 18.

The composition of this invention comprises one or more of polyethylene glycol, polyvinyl alcohol and polypropylene glycol and basal medium, and it may contain a low density lipoprotein, if desired.

As the basal medium, it may be any media which can be used for cultivating animal cells.

As the basal medium used in this invention, there may be mentioned, for example a variety of commercially available basal media such as Eagle's Minimum Essential Medium (MEM) (Science, 130: 432, 1959), Eagle's Basal Medium (BME) (Proceedings of the Society for Experimental Biology and Medicine, 89: 362, 1965), Dulbecco's Modified Eagle Medium (DME) (Virology, 8: 396, 1959), Iscove's Modified Dulbecco's Medium (IMDM) (The Journal of Experimental Medicine, 147: 923, 1978), Leibovitz's L-15 Medium (American Journal of Hygiene, 78: 173, 1963), McCoy's 5a Medium (Proceedings of the Society for Experimental Biology and Medicine, 100: 115, 1959), Ham's F12 Medium (Proceedings of National Academy of Science, USA, 53: 288, 1965), RPMI 1640 Medium (Journal of the American Medical Association, 199: 519, 1967) or the like, or a mixed medium thereof. Furthermore, there are mentioned serum-free media comprising the above-mentioned basal media to which have been added, if necessary, substances essential for growing respective cells (supplementary growth factors), for example, hormones such as insulin, transferrin, steroid hormones and the like; proteinous growth factor such as epidermal growth factor (EGF), platelet derived growth factor (PDGF), fibroblast growth factor (FGF) and the like; heavy metals such as sodium selenite and the like; and phospholipids such as ethanolamine, phosphatidylethanolamine and the like.

Furthermore, the basal media may be those to which have been added serum substitutes such as GFS (Text for 2nd Symposium on Research and Development Project of Basic Technology for Future Industries, 161, 1984), NU-SERUM (manufactured by Collaborative Research, Inc.), SERUM PLUS (manufactured by KC Biological Co.) and the like in a usual amount or less, or those to which have been added serum such as fetal calf serum (FCS), new born calf serum, calf serum, swine serum, goat serum, chicken serum, etc. in an amount less than usual amount used. There may be also used commercially available serum-free media [e.g. Hybrity-1 (manufactured by Nihon Yakuhin Kaihatsu Co.), HB-102 (manufactured by Hana Media, Inc.), HL-1 (manufactured by Ventrex) and the like] as the basal medium, or there may be also prepared a medium of which the concentration of an amino acid or the like has been adjusted to an optimal concentration according to that of a commercially available medium.

The polyethylene glycol used in this invention is a high polymer having a construction of $[-CH_2CH_2O-]_n$ (wherein n represents a polymerization degree), preferably a high polymer having a mean molecular weight of about 1000 or more, among which is preferably a high polymer having a mean molecular weight of about 2,000 - 20,000, particularly, having a mean molecular weight of about 4,000, about 6,000 or about 20,000. The polyethylene glycol used in this invention is preferably used in a concentration less than 10% (W/V). The term "less than" herein used means exclusive of "zero". The amount of polyethylene glycol is such an amount that the concentration on use is more preferably in the range of about 0.001 - 10% (W/V), further preferably in the range of about 0.01 - 2% (W/V).

The polyvinyl alcohol used in this invention is a high polymer having a construction of $[-CH_2CH(OH)-]_{n'}$ - (wherein n' represents a polymerization degree), preferably a high polymer having a mean polymerization degree of about 500 - 2,000, particularly about 500, and in the range of 1,500 - 1,800.

The polyvinyl alcohol used in this invention is preferably used in an concentration of about 0.001 - 10% (W/V), more preferably about 0.01 - 3% (W/V).

The polypropylene glycol used in this invention includes polypropylene glycols of diol or triol type and the like, preferably those of triol type. The mean molecular weight is preferably in the range of about 3,000 - 4,000, particularly about 4,000. The term of "diol type" refers to a high polymer having a construction of

$$HO[-CH(CH_3)CH_2O-]_{n_1}H,$$

(wherein $n_1$ represents a polymerization degree). On the other hand, the polypropylene glycols of triol type include, for example, high polymers which have a construction of $ROCH_2CH(OR)CH_2OR$ and a mean molecular weight of 3,000, those which have a construction of $CH_3CH_2C(CH_2OR)_3$ and a mean molecular weight of 4,000 or the like, wherein R refers to

$$[-CH_2CH(CH_3)O-]_{n_2}H$$

and $n_2$ represents a polymerization degree.

The polypropylene glycol used in this invention is preferably used in a concentration of about 0.001 - 1% (W/V), particularly in a concentration of about 0.01 - 0.1% (W/V).

The low density lipoprotein used in this invention is a lipoprotein which is separated from animal plasma, has a specific gravity of 1.019 - 1.063 and a molecular weight of (2.2 - 2.3) x $10^6$ and forms particle having a diameter of 19 - 25 nm. The lipoprotein comprises lipids, carbohydrates and proteins and plays a role of supplying cholesterol to cells in an organism.

In this invention, there may be used a commercially available LDL (e.g. human LDL, bovine LDL, etc.) or an LDL prepared from an animal serum such as bovine or swine serum, etc. according to the methods which are disclosed, for example, in "Cell Culture Methods for Molecular and Cell Biology", Alan R. Liss, Inc., New York, 1: 69, 1984 or will be illustrated hereunder in a referential example or the like. The LDL prepared according to the method in the referential example is used particularly advantageously.

The LDL used in this invention is preferably used in an amount of about 0.01 - 1000 µg/ml, particularly about 0.05 - 500 µg/ml, and more preferably about 0.5 - 100 µg/ml. In this specification, the amount of the LDL is represented in terms of the amount of protein which is contained in the LDL.

Polyethylene glycol, polyvinyl alcohol and polypropylene glycol may be used respectively alone or simultaneously in combination of the two or three. The incorporating ratio on using simultaneously in combination thereof is preferably in the range of about 0.1 - 100 by weight of PVA, more preferably about 0.1 - 10 by weight, most preferably about 0.5 - 2 by weight in terms of PEG as 1. On the other hand, the ratio of PEG is preferably in the range of about 1 - 100 by weight and that of PVA is in the range of about 0.5 - 100 by weight in terms of PPG as 1. More preferably PEG is incorporated in a proportion of about 1 - 20 by weight and PVA in a proportion of about 1 - 20 by weight.

The low density lipoprotein may be incorporated in either one of polyethylene glycol, polyvinyl alcohol or polypropylene glycol or in the combination thereof.

PEG, PVA, PPG and LDL used in this invention may have previously been incorporated in the composition or may be incorporated on use as a medium.

The composition for cultivating animal cell of this invention may be either solid or liquid. The solid composition is used by dissolving or suspending it into, for example in water.

The composition can be used as a medium for cultivating animal cell. When it is used as a culture medium, it may be formed as a serum-free medium or as a medium containing serum in an amount less than usual amount used. The composition is usually used in an amount of, for example, about 10% (V/V).

When the composition of this invention is used as a medium, it may be a medium containing a serum substitute in an amount for usual use or less. In this place, the amount of the substitute usually used is about 3 - 4 g/l (in terms of protein) for GFS, about 10% (V/V) for NU-SERUM and about 10% (V/V) for SERUM PLUS.

The animal cell to be cultivated according to the process of this invention is not limited. As the examples thereof, there can be mentioned lymphocytic cells derived from mammals such as human, mouse, rat, bovine, hamster and the like, such as normal lymphocyte, myeloma cell, B-lymphoblastoid cell, T-lymphocytic leukemia cell and the like, a variety of hybridomas such as mouse hybridoma, mouse-human heterohybridoma, human hybridoma and the like, normal dipoid cells such as fibroblast and the like, a variety of other adherent cells and the like.

More particularly, the human lymphocytic cells include Namalva ATCC CRL 1432 (Human lymphoblastoid, Burkitt lymphoma) (International Journal of Cancer, 12: 396-408, 1973); Raji ATCC CCL 86 (Burkitt lymphoma, Human) (Lancet, 1: 238, 1964); EB-3 ATCC CCL 85 (Burkitt lymphoma, Human) (Lancet, 1: 252, 1964); WI-L2 (Cancer, 22: 517, 1968); Daudi ATCC CCL 213 (Burkitt lymphoma, Human) (Cancer Research, 28: 1300-1310, 1968); RPMI 8226 ATCC CCL 155 (Myeloma, Human) (Proceedings of the Society for Experimental Biology and Medicine, 125: 1246-1250, 1967); CCRF-CEM ATCC CCL 119 (Peripheral blood, Human; Acute lymphoblastic leukemia) (Cancer, 18: 522-529. 1965); RPMI 1788 ATCC CCL 156 (Peripheral blood, HUMAN) (IgM secreting) (Journal of the National Cancer Institute (United

States), 43: 1119-1128, 1969); CCRF-SB ATCC CCL 120 (Peripheral blood, Human; Acute lymphoblastic leukemia) (Cancer Research, 27: 2479-2482, 1967); Jurkat (Immunogenetics, 10: 247, 1980) and the like. Mouse lymphocytic cells include, for example, MPC-11 ATCC CCL 167 (Myeloma, Mouse) (Immunoglobulin secreting) (The Journal of Experimental Medicine, 131: 515-541, 1970); P3/NSI/I-Ag4-I (NS-I) ATCC TIB 18 (Non-secreting mouse myeloma) (European Journal of Immunology, 6: 511, 1976); P3X63Ag8U.I (P3UI) ATCC CRY 1597 (Mouse myeloma) (Current Topics of Microbiology and Immunology, 81: 1-7, 1978) and the like.

Hybridomas include, for example, mouse hybridoma CEA (Text for 2nd Symposium on Research and Development Project of Basic Technology for Future Industries, 175, 1984), HS-II (Ibid.), E235I63 (Hybridoma, 4. 47, 1985), mouse•human-human heterohybridoma N12-16.63 (Text for 2nd Symposium on Research and Development Project of Basic Technology for Future Industries, 175, 1984), high productive strains derived from N12-16.63 such as N12-16.63.49.19, N12-16.63.49.19.69 and the like (Text for 3rd Symposium on Research and Development Project of Basic Technology for Future Industries, 155, 1985), I12-22.25 (Biochemical and Biophysical Research Communication, 129: 26, 1985); HB III-43.1 (Ibid), and the like. The adherent cells include, for example, FL ATCC CCL 62 (Amnion, Human, HeLa Markers), (Proceedings of the Society for Experimental Biology and Medicine, 94: 523, 1957); HeLa ATCC CCL 2 (Epitheloid carcinoma, cervix, Human) (Cancer Research, 12: 264, 1952); WISH ATCC CCL 25 (Amnion, Human, HeLa, Markers) (Experimental Cell Research, 23: 14, 1961); CHO-K1 ATCC CCL 61 (Ovary, Chinese hamster, Cricetulus griseus) (The Journal of Experimental Medicine, 108: 945, 1958); NCTC clone 929 ATCC CCL 1 (Connective tissue, Mouse), Clone of strain L (Journal of National Cancer Institute (U.S.A.), 9: 229, 1948) and the like.

As the biologically active substance producing animal cells which are used in the process for producing the biologically active substance of this invention, there are mentioned CEA, HS-II, E235163 and the like which produce mouse monoclonal antibody; N12-16.63 and a mutant thereof, I12-22.25, HB III-43.1 and the like which produce human monoclonal antibody; Namalva cell which produces leukocyte interferon; Jurkat cell which produces interleukin-2; genetic recombinant cells [mouse L-IL213-3 cell, human FL-IL385-6 cell, hamster C-IL485-14 cell (see Japanese Patent Unexamined Publication No. 63282/86) and the like]; etc.

For the culture according to the process of this invention, there are used vessels or apparatuses which are used for usual cultivation. For example, in the case of suspended cell culture, a multi-well plate, a culture flask, a spinner flask, a jar fermentor, a fermentor or the like is used, and in addition, an apparatus such as a hollow fiber bioreactor, a culture apparatus using ceramic matrix or the like and a culture method with microcapsules or the like are employed appropriately. In the case of adherent cells, a multi-well plate, a culture flask, a roller bottle, the microcarrier culture method, the hollow fiber culture method, the ceramic matrix culture method and the like are used.

In the culture of this invention, conditions suitable to the culture of the animal cell used are employed. In general, culture is carried out at a culture temperature of about 37°C at a pH of about 6.5 - 7.5 for a period from several days to three months. For example, if cells are usually inoculated in the medium of this invention in an amount of 0.1 - 5 x $10^5$/ml and a multi-well plate or a flask are used, incubation is carried out at about 37°C at a pH of about 6.5 - 7.5 in a 5% carbon dioxide incubator (an incubator having a carbon dioxide concentration of 5%) for a period from about 1 day to 20 days. Furthermore, if culture is carried out using these incubators, a hollow fiber incubator, a ceramic matrix incubator, a microcapsule incubator or the like, the productivity of the bilogically active substance can be improved by replacing batchwise or continuously the media. When perfusion culture is carried out, it sometimes continues for a period from one to several months (about 3 months). The medium are aerated, if necessary.

In order to collect cells, e.g. suspended cells, from the cultured medium, it is in situ subjected to centrifugation or filtration to collect the cells. In the case of adherent cells, 0.1 mg/ml of EDTA and 1.25 mg/ml of trypsin are added to the medium and the mixture is reacted at 37°C for 1 - 2 minutes to separate the cells, which are then collected by centrifugation or filtration.

When the biologically active substance produced by cell culture is accumulated in the cultured medium, filtration or centrifugation gives the supernatant, from which the substance is collected. When the substance is accumulated within cells, the cells obtained by filtration or centrifugation is treated by a physical method such as ultrasonic, French press, Dyno®-MILL (manufactured by Willy A. Bachofen AG) or the like or a chemical method for example with guanidine hydrochloride or the like to extract a product, and then the supernatant is obtained.

In order to separate and purify the biologically active substance from the aforementioned supernatant, it is possible to combine suitably separation and purification methods per se well known. For example, when the biologically active substance is protein or peptide, there may be applied the methods utilizing solubility such as salting-out, solvent precipitation or the like; the methods mainly utilizing the difference of molecular

weights such as dialysis, ultrafiltration, gel filtration, SDS-polyacrylamide gel electrophoresis or the like; the methods which uses the difference of electric charges such as ion exchange chromatography or the like; the methods which uses the specific affinity such as affinity chromatography or the like; the methods utilizing the difference of hydrophobicities such as reversed phase high performance liquid chromatography or the like; and the methods utilizing the difference of isoelectric points such as isoelectric point electrophoresis or the like.

The animal cells cultivated according to the process of this invention are utilized for the production of vaccines by infecting a virus or for the production of biologically active substances such as a variety of lymphokines (e.g. interferons, interleukins and the like), a variety of growth factors (e.g. epidermal growth factor, fibroblast growth factor and the like), hormones (e.g. human growth hormone, insulin and the like), enzymes (e.g. urokinase, tissue plasminogen activator and the like), a variety of monoclonal antibodies (e.g. mouse monoclonal antibody, human monoclonal antibody and the like), and the like. Further, it is also possible to produce efficiently the substances by using the cells into which specific gene has been introduced by gene manipulation. The cells grown and collected according to the process of this invention can be also utilized in situ as an artificial skin or an artificial organ (e.g. pancreatic beta islet cell, liver cell or the like). It is also possible to use normal lymphocytes for adoptive immunotherapy by growing them by stimulating with interleukin-2 and returning the collected cells directly into human blood.

According to the process of this invention, animal cells can be grown efficiently, and thus the process of this invention can be used advantageously as a method for growing industrially animal cells in a large amount.

When the biologically active substance producing animal cells are grown according to the process of this invention, the cells are efficiently grown, so that a biologically active substance can be produced efficiently. Thus the process has an advantage industrially.

## Examples

This invention is specifically explained below with reference to Referential Example and Examples, but the invention is not limited thereto. The percentage of addition of serum substitutes except GFS and serum is in terms of V/V %, and the percentages of PEG, PVA and PPG added are in terms of W/V %. The amount of LDL added is represented as an amount of protein contained in LDL.

Human LDL used in Examples are an LDL commercially available from Sigma Chemical Co. and swine LDL is an LDL produced by the process illustrated by Referential Example 1.

### Referential Example 1

One liter of swine serum was dialyzed against 10 mM tris-hydrochloric acid buffer (pH 7.2) to which 5 mM EDTA and 1M NaCl had been added and charged in a Cibacron Blue F3G-A column (manufactured by Bio-Rad; 2.5 cm ⌀ x 18 cm) equilibrated with the same buffer as above. The column was sufficiently washed with the same buffer as above, and then content was eluted with 300 ml of an eluent which was the buffer having been added thereto 0.5 M NaSCN. The active fraction was collected, and a low specific gravity solution (0.15 M NaCl, d = 1.006) was added to the fraction to adjust the specific gravity to d = 1.019. The mixture was centrifuged at 35000 rpm for 30 hours. About 100 ml of the bottom layer was collected and a high specific gravity solution (d = 1.346, 153.0 g of NaCl and 354.0 g of KBr were dissolved in water to a final volume of 1 liter) was added to the bottom layer to adjust the specific gravity to d = 1.069. The mixture was subjected to ultra-centrifugation (35000 rpm, 30 min., 4 °C) and about 10 ml of LDL layer (uppermost layer, containing about 10.6 mg of LDL) was obtained.

### Example 1

To a medium in which IMDM, Ham's F12 Medium and L-15 Medium had been mixed in a ratio (volume) of 1:1:2 were added 2 mg/l of insulin, 2 mg/l of transferrin, 2 x $10^{-6}$ M of ethanolamine, 2 x $10^{-8}$ M of sodium selenite (the combination of these four referred to as ITES). Media were prepared by adding to the above mixture a various concentrations of PEG's having a variety of mean molecular weights (PEG 400, PEG 1000, PEG 1540, PEG 2000, PEG 4000, PEG 6000 and PEG 20000) and distributed into multi-well plates having 24 wells in an amount of 1 ml, respectively. To the plate was inoculated the cell of mouse•human-human heterohybridoma N12-16.63 strain in a level of 1x$10^5$/ml. After culture was carried out at 37 °C for 5 days in a 5% carbon dioxide incubator, the number of cells were counted with a Coulter Counter®.

The results are illustrated in Fig. 1. In Fig. 1, the graph of -⊙- shows the number of cells on adding PEG20,000 in the concentrations shown in Fig. 1. Similarly, the graph of -○- shows the number of cells on adding PEG 6,000, -▲- on adding PEG 4,000, -△- on adding PEG 2,000, -■- on adding PEG 1,540, -□- on adding PEG 1,000, and -●- on adding PEG 400, respectively.

Apparently from Fig. 1, while the number of the cells in the medium without adding PEG thereto was 2.5 x $10^5$/ml, the number of the cells were increased along with the increase of concentration of PEG except PEG400 when PEG was added. The optimal concentration was present at the concentration of about 0.25% of PEG, but the effect adding PEG was observed even in such a low concentration of 0.001%. The effect was more remarkable as PEG has a larger mean molecular weight. In the case of PEG 20,000, the number of the cells reached 3.3 times of that obtained without adding PEG under the optimal condition.

Example 2

To the same basal medium as in Example 1 was added ITES in the same concentration. Media were prepared by adding to the above basal medium various concentrations of PVA (mean polymerization degree: 500) or PPG (triol type, mean molecular weight: 4,000) and distributed into multi-well plates in an amount of 1 ml, respectively. To the plate was inoculated the cell of mouse•human-human heterohybridoma N12-16.63 strain in a level of 1 x $10^5$/ml. After culture was carried out at 37°C for 5 days in a 5% carbon dioxide incubator, the number of cells were counted with a Coulter Counter®. The results were shown in Figs. 2 and 3.

The optimal concentration of PVA was in the range of 0.2 - 0.8%, and that of PPG was 0.025%. Under these optimal conditions, the number of the cells reached about 4.5 times of that obtained without adding PEG for PVA and about 3 times for PPG.

Example 3

To the same basal medium as in Example 1 was added ITES in the same concentration. Media were prepared by adding to the above basal medium PEG 20,000 (mean molecular weight: 20,000), PVA (mean polymerization degree: 500) and PPG (triol type, mean molecular weight: 4,000) alone or in combination thereof in the amounts shown in Table 1 below. To the media was inoculated the cell of mouse•human-human heterohybridoma N12-16.63 strain in a level of 1x$10^5$/ml. After culture was carried out at 37°C for 5 days in a 5% carbon dioxide incubator, the number of cells were counted with a Coulter Counter®. The results shown in Table 1 are obtained.

Table 1: Combination Effect of Respective Polymers

| Amount of polymer added (%) | | | Number of cells |
|---|---|---|---|
| PEG | PVA | PPG | |
| 0 | 0 | 0 | 2.0 x $10^5$ |
| 0.02 | 0 | 0 | 6.0 x $10^5$ |
| 0 | 0.02 | 0 | 5.2 x $10^5$ |
| 0 | 0 | 0.02 | 3.5 x $10^5$ |
| 0.02 | 0.02 | 0 | 8.5 x $10^5$ |
| 0.02 | 0 | 0.02 | 8.0 x $10^5$ |
| 0 | 0.02 | 0.02 | 7.5 x $10^5$ |

Example 4

To the same basal medium as in Example 1 was added ITES in the same concentration. Two media were prepared, one of which was prepared by adding to the above basal medium 0.1% of PEG 20,000 (mean molecular weight: 20,000) and the other contained no PEG 20,000 and distributed into multi-well plates having 24 wells in an amount of 1 ml, respectively. After human LDL (manufactured by Sigma Chemical Co.) was added to each of the wells, the cell of mouse•human-human heterohybridoma N12-16.63.49.19 strain was inoculated in a level of $1x10^5$/ml. After culture was carried out at 37°C for 5 days in a 5% carbon dioxide incubator, the number of cells were counted with a Coulter Counter®. The results were shown in Fig. 4. In Fig. 4, the graph of -o- represents the number of cells on adding only human LDL, and the graph of -•- represents the number of cells on adding simultaneously the human LDL and 0.1% PEG 20,000.

Apparent from Fig. 4, when 0.1% PEG and the human LDL were added at the optimal concentration (3.12 to 25 µg/ml), the growth of the cell was remarkably increased as compared with the cases of adding only one of 0.1% PEG or the human LDL at the optimal concentration.

Example 5

In place of the human LDL in Example 4, the swine LDL prepared in accordance with Referential Example 1 was added in various concentrations, the treatment was carried out in the same manner as in Example 4 to count the number of the cells. The results are shown in Fig. 5. In Fig. 5, the graph of -o- represents the number of cells on adding only swine LDL, and the graph of -•- represents the number of cells on adding simultaneously the swine LDL and 0.1% PEG 20,000.

Apparent from Fig. 5, when 0.1% PEG and the swine LDL were added at the optimal concentration (0.36-200 µg/ml), the growth of the cell was remarkably increased as compared with the cases of adding only one of 0.1% PEG or the swine LDL at the optimal concentration.

When Figs. 4 and 5 are compared with each other, the swine LDL prepared in accordance with the process of Referential Example 1 has apparently greater synergistic effect with PEG compared with the commercially available human LDL (manufactured by Sigma Chemical Co.) and shows stable growth in a wide range of concentration.

Example 6

To the same basal medium as in Example 1 was added ITES in the same concentration. To this medium (1) was added further 0.025% PPG (triol type, mean molecular weight: 4,000), 2 µg/ml of human LDL (manufactured by Sigma Chemical Co.), and 0.025% PPG and 2 µg/ml of human LDL to prepare media (2), (3) and (4), respectively. These media were distributed into multi-well plates having 24 wells in an amount of 1 ml, respectively. To the plate was inoculated the cell of mouse•human-human heterohybridoma N12-16.63.49.19.69 strain in a level of $1x10^5$/ml. After culture was carried out at 37°C for 3 days in a 5% carbon dioxide incubator, the number of cells were counted with a Coulter Counter®. The results are shown in Table 2.

Apparently from Table 2, when 0.025% PPG and 2 µg/ml of human LDL were added, the growth of the cell was remarkably increased as compared with the cases of adding only one of 0.025% PPG or 2 µg/ml of human LDL.

Table 2: Combination Effect of PPG and LDL

| Medium No. | PPG | LDL | Number of Cells |
|---|---|---|---|
| (1) | 0 | 0 | $1.1 \times 10^5$ |
| (2) | 0.025% | 0 | $1.8 \times 10^5$ |
| (3) | 0 | 2 μg/ml | $1.5 \times 10^5$ |
| (4) | 0.025% | 2 μg/ml | $3.1 \times 10^5$ |

Example 7

To the same basal medium as in Example 1 was added ITES in the same concentration. To this medium (1) was added further 0.25% PEG 20,000 or 0.25% PEG 2,000 to prepare media (2) or (3), respectively. These media were distributed into multi-well plates having 24 wells in an amount of 1 ml, respectively. To these plates were inoculated various lymphocytic cells shown in Table 3 in a level of 1 x $10^5$/ml, and culture was carried out at 37°C for 5 days in a 5% carbon dioxide incubator. Then, the number of cells were counted with a Coulter Counter®. The results were shown in Table 3. As shown in Table 3, the effect of addition was recognized extensively in the culture of various lymphocytes and various hybridomas.

Table 3: Effect of PEG on the growth of various lymphocytic cells in a serum-free medium

| Cell strain | Origin | No addition | Additon of PEG20,000 | Addition of PEG2,000 |
|---|---|---|---|---|
| EB-3 | Human Burkitt lymphoma | $5.8 \times 10^4$ | $3.0 \times 10^5$ | $2.9 \times 10^5$ |
| Namalva | Human Burkitt lymphoma | $4.9 \times 10^5$ | $1.5 \times 10^6$ | $1.5 \times 10^6$ |
| CCRF-SB | Human acute lymphoblastic leukemia | $5.8 \times 10^4$ | $4.9 \times 10^5$ | $4.7 \times 10^5$ |
| Daudi | Human Burkitt lymphoma | $6.7 \times 10^4$ | $5.3 \times 10^5$ | $5.5 \times 10^5$ |
| WI-L2 | Human lympho-blastoid | $8.5 \times 10^5$ | $2.6 \times 10^6$ | $2.5 \times 10^6$ |
| RPMI1788 | Human lympho-blastoid | $3.1 \times 10^5$ | $1.3 \times 10^6$ | $1.1 \times 10^6$ |
| E235I63 | Mouse hybridoma | $6.3 \times 10^5$ | $1.0 \times 10^6$ | $9.5 \times 10^5$ |
| I12-22.25 | Mouse·human-human hybridoma | $2.3 \times 10^5$ | $7.4 \times 10^5$ | $7.5 \times 10^5$ |

Example 8

To the same basal medium as in Example 1 was added ITES in the same concentration. To this medium (1) was added further 0.2% PVA (mean polymerization degree: 500) or 0.025% PPG (triol type, mean molecular weight: 4,000) to prepare media (2) or (3), respectively. These media were distributed into multi-well plates having 24 wells in an amount of 1 ml, respectively. To these plates were inoculated various lymphocytic cells listed on Table 4 in a level of $1 \times 10^5$/ml, and culture was carried out at 37°C for 5 days in a 5% carbon dioxide incubator. Then, the number of cells were counted with a Coulter Counter®. The results are shown in Table 4.

Table 4

| Effect of PVA or PPG on the growth of various lymphocytic cells in a serum-free medium | | | | | |
|---|---|---|---|---|---|
| Cell strain | Origin | No addition | Addition of PVA | Addition of PPG |
| WI-L2 | Human lymphoblastoid | $6.9 \times 10^5$ | $3.1 \times 10^6$ | $1.9 \times 10^6$ |
| Namalva | Human Burkitt lymphoma | $2.1 \times 10^5$ | $1.3 \times 10^6$ | $8.9 \times 10^5$ |
| CCRF-SB | Human acute lymphoblastic leukemia | $6.0 \times 10^4$ | $3.3 \times 10^5$ | $1.8 \times 10^5$ |
| P3UI | Mouse myeloma | $5.1 \times 10^4$ | $1.3 \times 10^5$ | $6.1 \times 10^5$ |
| E235I63 | Mouse hybridoma | $3.5 \times 10^5$ | $9.9 \times 10^5$ | $4.2 \times 10^5$ |
| N12-16.63 | Mouse•human-human heterohybridoma | $2.6 \times 10^5$ | $1.0 \times 10^6$ | $5.5 \times 10^5$ |

Example 9

To the same basal medium as in Example 1 was added ITES in the same concentration. To this medium (1) was added further 0.2% PVA (mean polymerization degree: 1500-1800) to prepare a medium (2). This medium was distributed into multi-well plates having 24 wells in an amount of 1 ml, respectively. To these plates were inoculated various lymphocytic cells listed on Table 5 in a level of $1 \times 10^5$/ml, and culture was carried out at 37°C for 4 days in a 5% carbon dioxide incubator. Then, the number of cells were counted with a Coulter Counter®. The results are shown in Table 5.

Table 5

| Effect of PVA on the growth of various cells in a serum-free medium | | | |
|---|---|---|---|
| Cell strain | Origin | No addition | Addition of PVA |
| WI-L2 | Human lymphoblastoid | $6.9 \times 10^5$ | $2.5 \times 10^6$ |
| Namalva | Human Burkitt lymphoma | $2.1 \times 10^5$ | $8.9 \times 10^5$ |
| CCRF-SB | Human acute lymphoblastic leukemia | $6.0 \times 10^4$ | $2.6 \times 10^5$ |
| E235I63 | Mouse hybridoma | $3.5 \times 10^5$ | $5.4 \times 10^5$ |
| N12-16.63 | Mouse•human-human heterohybridoma | $2.6 \times 10^5$ | $5.2 \times 10^5$ |

Example 10

To the same basal medium as in Example 1 was added ITES in the same concentration. To this medium (1) was added further 0.1% PEG 20,000, 0.1% PVA (mean polymerization degree: 500), 0.05% PEG 20,000 and 0.05% PVA (mean polymerization degree: 500) to prepare media (2), (3) and (4), respectively. To these media was added 10 µg/ml of swine LDL obtained in accordance with Referential Example 1 to prepare media (5) - (8), respectively. These media were distributed into multi-well plates having 24 wells in an amount of 1 ml, respectively. To these plates were inoculated various lymphocytic cells listed on Table 6 in a level of $1 \times 10^5$/ml, and culture was carried out at 37°C for 3 days in a 5% carbon dioxide incubator. Then, the number of cells were counted with a Coulter Counter®. The results are shown in Table 6.

Table 6: Effect of PEG, PVA and LDL on the growth of various cells

| Cell strain \ Medium No. | Number of cells ($x10^5$/ml) | | | | | | | |
|---|---|---|---|---|---|---|---|---|
| | (1) | (2) | (3) | (4) | (5) | (6) | (7) | (8) |
| I12-22.25 (Mouse·human-human heterohybridoma) | 1.0 | 2.7 | 2.9 | 2.7 | 1.1 | 4.8 | 4.9 | 4.9 |
| E235I63 (Mouse-mouse hybridoma) | 2.4 | 5.3 | 4.5 | 4.7 | 2.0 | 5.8 | 5.4 | 6.0 |
| Namalva (Human Burkitt lymphoma) | 0.3 | 1.6 | 2.3 | 2.4 | 0.6 | 3.3 | 3.9 | 3.9 |
| WI-L2 (Human lymphoblastoid) | 0.4 | 4.8 | 4.7 | 5.0 | 0.8 | 5.3 | 6.3 | 6.4 |

Apparently from the result shown in Table 6, a variety of cells are grown extensively by adding PEG and/or PVA and LDL.

Example 11

To the same basal medium as in Example 1 was added ITES in the same concentration. To this medium (1) was added further 0.1% PEG 20,000, 0.1% PVA (mean polymerization degree: 500), 0.05% PEG 20,000 and 0.05% PVA (mean polymerization degree: 500) to prepare media (2), (3) and (4), respectively. To these media was added 10 µg/ml of human LDL obtained in accordance with Referential Example 1 to prepare media (5) - (8), respectively. These media were distributed into multi-well plates having 24 wells in an amount of 1 ml, respectively. To these plates were inoculated various lymphocytic cells listed on Table 7 in a level of 1 x $10^5$/ml, and culture was carried out at 37°C for 3 days in a 5% carbon dioxide incubator. Then, the number of cells were counted with a Coulter Counter®. The results are shown in Table 7.

Table 7: Effect of PEG, PVA and LDL on the growth of various cells

| Cell strain \ Medium No. | Number of cells ($x10^5$/ml) (1) | (2) | (3) | (4) | (5) | (6) | (7) | (8) |
|---|---|---|---|---|---|---|---|---|
| I12-22.25 (Mouse·human-human heterohybridoma) | 1.0 | 2.7 | 2.9 | 2.7 | 1.3 | 4.1 | 4.3 | 4.1 |
| E235I63 (Mouse-mouse hybridoma) | 2.4 | 5.3 | 4.5 | 4.7 | 2.1 | 6.1 | 5.9 | 6.0 |
| Namalva (Human Burkitt lymphoma) | 0.3 | 1.6 | 2.3 | 2.4 | 0.3 | 4.0 | 4.3 | 4.2 |
| WI-L2 (Human lymphoblastoid) | 0.4 | 4.8 | 4.7 | 5.0 | 0.7 | 6.1 | 6.3 | 6.2 |

Apparently from the result shown in Table 7, a variety of cells are grown extensively by adding PEG and/or PVA and LDL.

Example 12

Using the 1:1 mixture of IMDM and Ham's F12 Medium as a basal medium, ITES in the same concentration as that in Example 1 and FCS and PEG 20,000 in the concentrations shown in Table 8 were added to prepare a medium. To this medium was inoculated adherent human FL cell (ATCC CCL 62) in a level of 1 x $10^5$/ml and culture was carried out at 37°C for 5 days in a 5% carbon dioxide incubation, the cells were separated by using trypsin and the number of cells were counted with a Coulter Counter®. As shown in Table 8, remarkable growth promoting effect of PEG was recognized even in a serum-free medium, and when used a medium containing 1% of serum and 0.2% of PEG20,000, the growth of cells was observed more extensively than in a medium containing 10% of serum.

Table 8: Effect of PEG on human FL cell

| Serum | PEG20,000 | Number of cells (per 1 ml) |
|---|---|---|
| 0 | 0 | $1 \times 10^5$ |
| 0 | 0.01% | $4.6 \times 10^5$ |
| 0 | 0.2% | $4.7 \times 10^5$ |
| 1% | 0 | $4.9 \times 10^5$ |
| 1% | 0.01% | $9.0 \times 10^5$ |
| 1% | 0.2% | $1.2 \times 10^6$ |
| 10% | 0 | $9.4 \times 10^5$ |

Example 13

Using as the basal medium a medium in which to the same base medium as in Example 1 had been added 35 mg/l of L-proline for adherent hamster CHO-K1 (ATCC CCL 61) and MEM for mouse L cell, respectively, ITES in the same concentration as in Example 1 and FCS and PEG 20,000 in the concentrations shown in Table 9 were added to prepare media. To these media were inoculated the cells in a level of 1 x $10^4$/ml and culture was carried out at 37° C for 5 days in a 5% carbon dioxide incubator. After incubation, the cells were separated by using trypsin and the number of cells were counted with a Coulter Counter®.

Table 9: Effect of PEG on rodent-derived cell

| Cell | Serum | PEG 20,000 | Number of cells (per 1 ml) |
|---|---|---|---|
| CHO-K1 | 1% | 0 | $2.1 \times 10^5$ |
| | 1% | 0.1% | $6.4 \times 10^5$ |
| | 1% | 0.2% | $6.7 \times 10^5$ |
| | 5% | 0 | $7.5 \times 10^5$ |
| L | 2% | 0 | $1.4 \times 10^5$ |
| | 2% | 0.1% | $2.3 \times 10^5$ |
| | 2% | 0.2% | $3.2 \times 10^5$ |
| | 10% | 0 | $2.0 \times 10^5$ |

Apparently from Table 9, in the CHO-K1 cell, growth of the cells were recognized to almost equal extent to that using a medium containing 5% of serum by adding 0.1% of PEG 20,000 to 1% of serum. In the L cell, when 0.2% of PEG 20,000 was added to 2% of serum, growth was more extensive than that using 10% of serum.

Example 14

To the same basal medium as in Example 1 was added 1% of fetal calf serum (FCS). To this serum containing medium was added 0.1% of PEG 20,000 to prepare a medium and a PEG-free medium. The cells shown in Table 10 were cultured under the same conditions as in Example 1, and the results shown in Table 10 were obtained. As a control medium, a medium comprising the same basal medium to which 10% of FCS had been added was used.

Table 10

| Effect of adding PEG on a medium containing a low concentration of serum | | | |
|---|---|---|---|
| Cell | No addition | Addition of PEG 20,000 | Control (10% FCS medium) |
| N12-16.63 | $5.9 \times 10^5$ | $9.6 \times 10^5$ | $1.1 \times 10^6$ |
| HB III-43.1 | $5.2 \times 10^5$ | $8.3 \times 10^5$ | $9.1 \times 10^5$ |

Example 15

To the same basal medium as in Example 1 was added 1% of fetal calf serum (FCS). To this serum containing medium was added 0.2% of PVA (mean polymerization degree: 500) or 0.025% PPG (triol type, mean molecular weight: 4,000) to prepare a medium and a PVA or PPG-free medium. The mouse•human-human heterohybridoma N12-16.63 or mouse hybridoma E235I63 cells were inoculated at a level of 1 x $10^5$/ml in these media, and culture was carried out at 37°C for 5 days in a 5% carbon dioxide incubator. The number of the cells was counted with a Coulter Counter®. The results shown in Table 11 were obtained.

Table 11

| Effect of adding PVA or PPG on a medium containing a low concentration of serum | | | |
|---|---|---|---|
| Cell | No addition | Addition of PVA | Addition of PPG |
| N12-16.63<br>E235I63 | $5.9 \times 10^5$<br>$6.2 \times 10^5$ | $9.2 \times 10^5$<br>$1.6 \times 10^6$ | $7.8 \times 10^5$<br>$8.3 \times 10^5$ |

Example 16

To the same base medium as in Example 1 were added ITES in the same concentration as in Example 1 and a serum substitute GFS [sedimentation fraction of bovine serum with 55 - 70% (by saturation degree) of ammonium sulfate) in an amount of 0.3 mg/ml (1/10 of the amount usually used) as protein concentration. From the base medium containing ITES and GFS, media respectively containing no PEG 20,000 (1) and 0.1% of PEG 20,000 (2) were prepared. To these media was inoculated the mouse•human-human heterohybridoma N12-16.63 or HB III-43.1 cell in an amount of $1 \times 10^5$/ml, culture was carried out at 37°C for 5 days in a 5% carbon dioxide incubator. The results shown in Table 12 were obtained. In addition, as a control, the same basal medium as in Example 1 to which ITES in the same concentration as in Example and GFS in an amount of 3 mg/ml have been added was used.

Table 12

| Effect of PEG on a medium having been added thereto a low concentration of serum substitute | | | |
|---|---|---|---|
| Cell | Addition of 1/10 GFS | Addition of 1/10 GFS + PEG | Addition of GFS (Control) |
| N12-16.63<br>HB III-43.1 | $3.6 \times 10^5$<br>$1.8 \times 10^5$ | $8.2 \times 10^5$<br>$6.8 \times 10^5$ | $9.8 \times 10^5$<br>$8.0 \times 10^5$ |

Example 17

A variety of commercially available serum-free media (Hybrity-1, HB-102 and HL-1) and a medium made from a base medium having the same composition as that in Example 1 to which basal medium had been added either one of the commercially available serum substitute (NU-SERUM, SERUM PLUS) in an amount of 10% were provided. The growth of the mouse•human-human heterohybridoma N12-16.63 was examined on either cases of adding and not adding 0.1% of PEG 20,000 to the above-mentioned media, and the results shown in Table 13 were obtained.

Table 13: Effect of adding PEG in various commercially available media

| Medium | Addition of not PEG | Addition of PEG 20,000(*1) |
|---|---|---|
| Hybrity-1 | 100 | 169 |
| HB102 | 100 | 143 |
| HL-1 | 100 | 153 |
| Same basal medium as in Example 1 + NU-SERUM | 100 | 150 |
| Same basal medium as in Example 1 + SERUM PLUS | 100 | 132 |

(*1): Relative value in terms of the growth degree of PEG-free medium as 100.

Example 18

A medium was prepared by adding to the same basal medium as in Example 1 ITES in the same concentration as in Example 1 and 0.1% of PEG 20,000. To the medium were inoculated the WI-L2 or Namalva cells at a level of 1 x $10^5$/ml, and the mixtures were cultured at 37°C in a 5% carbon dioxide incubator. After 5 days of culture, the cells were transferred to a new medium so as the number of cell to be equal to that in old medium, and the culture was further continued. The results were shown in Fig. 6. In Fig. 6, the graph o-o represents the growth of the WI-L2 cells, and o-● represents the growth of the Namalva cells, respectively. As shown in Fig. 6, it was found that either WI-L2 and Namalva cells were grown quitely normally even after fifth subculture and thus the medium can be used for maintaining subculture of cells.

Example 19

A medium was prepared by adding to the same basal medium as in Example 12 ITES in the same concentration as in Example 1 and PEG 20,000 in a concentration shown in Table 14. To the medium was inoculated the mouse hybridoma E235I63 strain at a level of 1 x $10^5$/ml, and the mixture were cultured at 37°C for 4 days in a 5% carbon dioxide incubator. The number of cells and the amount of antibody produced were measured, and the results shown in Table 14 were obtained.

Table 14: Effect of PEG on the production of antibody by the mouse hybridoma

| Concentration of PEG 20,000 | Cell number | Amount of antibody produced(*) |
|---|---|---|
| 0 | $4.8 \times 10^5$ | 100 |
| 0.1% | $1.0 \times 10^6$ | 253 |
| 0.25% | $1.2 \times 10^6$ | 268 |

(*): Relative value in terms of the amount of antibody produced on adding no PEG 20,000 as 100.

Example 20

A medium was prepared by adding to the same basal medium as in Example 1 ITES in the same concentration as in Example 1 and PEG 20,000 in a concentration shown in Table 15. To the medium was inoculated the mouse•human-human heterohybridoma I12-22.25 strain at a level of $1 \times 10^5$/ml, and the mixtures were cultured at 37°C for 6 days in a 5% carbon dioxide incubator. The number of cells and the amount of antibody produced were measured, and the results shown in Table 15 were obtained.

Table 15: Effect of PEG on the production of human antibody by mouse·human-human heterohybridoma

| Concentration of PEG 20,000 | Cell number | Amount of antibody produced(*) |
|---|---|---|
| 0 | $2 \times 10^5$ | 100 |
| 0.1% | $6 \times 10^5$ | 280 |
| 0.25% | $7 \times 10^5$ | 330 |

(*): Relative value in terms of the amount of monoclonal antibody produced on adding no PEG 20,000 as 100.

To one liter of the supernatant of the cell cultures grown in the media to which no PEG 20,000 and 0.1% of PEG had been added was added ammonium sulfate, and the fractions sedimented with 37% - 50% (saturated degree) of the ammonium salt were collected. After dialysis, the product was charged on a Whatman DE52 column which had been equilibrated with 0.02M tris-hydrochloric acid buffer (pH 7.9) having been added thereto 0.07M NaCl, and the fractions passing through column without harboring were collected. The fractions were dialyzed and lyophilized and then dissolved in 35 ml of 0.05M MES buffer (pH 6.0) containing 0.005M NaCl. The solution was subjected to FPLC® (manufactured by Pharmacia) using Mono S HR 5/5 Column (manufactured by Pharmacia). The column was washed with the same buffer and then eluted under linear gradient with a 0.05M MES buffer (pH 6.0) containing 1M NaCl. The human monoclonal antibody was obtained in an amount of 1018 μg from the medium to which no PEG had been added and 3135 μg from the medium to which the PEG had been added, respectively.

Example 21

A medium was prepared by adding to the same basal medium as in Example 1 ITES in the same concentration as in Example 1 and PVA (mean polymerization degree: 500) or PPG (triol type, mean molecular weight: 4,000) in a concentration shown in Table 16. To this medium was inoculated the mouse•human-human heterohybridoma I12-22.25 strain at a level of 1 x $10^5$/ml, and the mixture was cultured at 37°C for 6 days in a 5% carbon dioxide incubator. The number of cells and the amount of antibody produced were measured. The results are shown in Table 16.

Table 16: Effect of PVA or PPG on the production of human monoclonal antibody by mouse•human-human heterohybridoma

| Substance added | Number of cells | Amount of antibody produced(*) |
|---|---|---|
| No addition | 2 x $10^5$ | 100 |
| 0.2% PVA | 6 x $10^5$ | 260 |
| 0.025% PPG | 4 x $10^5$ | 150 |

(*): Relative value in terms of the amount of antibody produced in a medium having no addition as 100.

To one liter of the supernatant of the cell cultures grown in respective media was added ammonium sulfate, and the fractions sedimented with 37% - 50% (saturated degree) of the ammonium salt were collected. After dialysis, the product was charged on a Whatman DE52 column which had been equilibrated with 0.02M tris-hydrochloric acid buffer (pH 7.9) having been added thereto 0.07M NaCl, and the fractions passing through column without harboring were collected. The fractions were dialyzed and lyophilized and then dissolved in 35 ml of 0.05M MES buffer (pH 6.0) containing 0.005M NaCl. The solution was subjected to FPLC® using Mono S HR 5/5 Column (manufactured by Pharmacia). The column was washed with the same buffer and then eluted under linear gradient with a 0.05M MES buffer (pH 6.0) containing 1M NaCl. When the fractions of IgG were collected, human monoclonal antibody was obtained only in an amount of 980 μg from the medium having no addition as a control, while from the media containing PVA and PPG, respectively, obtained the antibody in an amount of 2400 μg and 1520 μg.

Example 22

To the same basal medium as in Example 1 was added ITES in the same concentration as in Example 1 and further 0.1% of PEG 20,000, 10 μg/ml of human LDL, and 0.1% PEG 20,000 and 10 μg/ml of human LDL to prepare media (1), (2) and (3), respectively. These media were respectively charged into a 100 ml spinner flask. To these flasks was inoculated mouse•human-human heterohybridoma N12-16.63.49.19.69 strain at a level of 1 x $10^5$/ml, and culture was carried out with a rotation of 25 rpm at 37°C for 6 days. Then, the number of cells and the amount of antibody produced were measured. The results are shown in Table 17.

Table 17: Effect of PEG and LDL on the production of antibody by mouse·human-human heterohybridoma

| Culture medium | Number of cells | Amount of antibody produced |
|---|---|---|
| (1) | 1.8 x $10^5$ | 10 μg/ml |
| (2) | 2.5 x $10^5$ | 16 μg/ml |
| (3) | 7.5 x $10^5$ | 62 μg/ml |

To one liter of the supernatant of the cell cultures grown in the above-mentioned medium (3) was added ammonium sulfate, and the fractions sedimented with 0% - 45% (saturated degree) of the ammonium salt were collected. After dialysis against 5 mM tris-hydrochloric acid buffer (pH 7.5) containing 5 mM of EDTA, the resulting sedimentation was collected and dissolved in 3.2 ml of 50 mM tris-hydrochloric acid (pH 8.0) containing 5 mM of EDTA and 0.5M NaCl. The solution was charged on a Sephacryl S-300 gel column (about 100 ml) which had been equilibrated with the same buffer and the active fractions were collected. Antitetanus toxoid human IgM was obtained in an amount of about 13 mg.

EFFECTS OF THE INVENTION

The composition for growing animal cell of this invention exhibits the cell proliferation activity over a wide range of animals even in serum-free state, and thus it is possible to grow efficiently animal cells in a large scale by using the medium according to this composition. Also, when the biologically active substance producing animal cell is cultured, the cell is efficiently proliferated in a large scale, so that it is possible to produce efficiently a biologically active substance.

**Claims**

1. A serum-free composition for promoting growth of animal cells which contains one or more of polyethylene glycol in a concentration of 10% (w/v) or less, polyvinyl alcohol and polypropylene glycol, wherein said composition contains a low density lipoprotein.

2. A composition according to claim 1, wherein said composition contains polyethylene glycol.

3. A composition according to claim 1, wherein said composition contains polyethylene glycol and/or polyvinyl alcohol and a low density lipoprotein.

4. A composition according to any of claims 1 to 3, wherein said composition is a solid.

5. A composition according to any of claims 1 to 3, wherein said composition is a solution.

6. A process for growing animal cells, comprising growing animal cells in a serum-free medium containing one or more of polyethylene glycol in a concentration of 10% (w/v) or less, polyvinyl alcohol and polypropylene glycol, wherein said medium contains a low density lipoprotein.

7. A process according to claim 6, wherein the medium for growing the animal cells contains polyethylene glycol.

8. A process according to claim 6, wherein the medium for growing the animal cells contains polyethylene glycol and/or polyvinyl alcohol and a low density lipoprotein.

9. A process for producing a biologically active substance, comprising growing a biologically active substance-producing animal cells in a serum-free medium containing one or more of polyethylene glycol in a concentration of 10% (w/v) or less, polyvinyl alcohol and polypropylene glycol and in addition, if desired, a low density lipoprotein, producing and accumulating said substance in the culture and collecting the substance, wherein said medium contains a low density lipoprotein.

**Patentansprüche**

1. Serumfreie Zusammensetzung zur Förderung des Wachstums tierischer Zellen, enthaltend eine oder mehrere der Komponenten Polyethylenglycol in einer Konzentration von 10 % (Gew./Vol.) oder weniger, Polyvinylalkohol und Polypropylenglycol, worin die Zusammensetzung ein Lipoprotein niedriger Dichte enthält.

2. Zusammensetzung nach Anspruch 1, worin die Zusammensetzung Polyethylenglycol enthält.

3. Zusammensetzung nach Anspruch 1, worin die Zusammensetzung Polyethylenglycol und/oder Polyvinylalkohol und ein Lipoprotein niedriger Dichte enthält.

4. Zusammensetzung nach irgendeinem der Ansprüche 1 bis 3, worin die Zusammensetzung ein fester Stoff ist.

5. Zusammensetzung nach irgendeinem der Ansprüche 1 bis 3, worin die Zusammensetzung eine Lösung ist.

6. Verfahren zum Kultivieren tierischer Zellen, umfassend das Wachsenlassen tierischer Zellen in einem serumfreien Medium, das eine oder mehrere der Komponenten Polyethylenglycol in einer Konzentration von 10 % (Gew./Vol.) oder weniger, Polyvinylalkohol und Polypropylenglycol enthält, worin das Medium ein Lipoprotein niedriger Dichte enthält.

7. Verfahren nach Anspruch 6, worin das Medium zum Wachsenlassen der tierischen Zellen Polyethylenglycol enthält.

8. Verfahren nach Anspruch 6, worin das Medium zum Wachsenlassen der tierischen Zellen Polyethylenglycol und/oder Polyvinylalkohol und ein Lipoprotein niedriger Dichte enthält.

9. Verfahren zum Produzieren einer biologisch aktiven Substanz, umfassend das Wachsenlassen tierischer Zellen, die eine biologisch aktive Substanz erzeugen, in einem serumfreien Medium, das eine oder mehrere der Komponenten Polyethylenglycol in einer Konzentration von 10 % (Gew./Vol.) oder weniger, Polyvinylalkohol und Polypropylenglycol und zusätzlich, falls gewünscht, ein Lipoprotein niedriger Dichte enthält, das Produzieren und Anreichern der genannten Substanz in der Kultur und das Sammeln der genannten Substanz, worin das Medium ein Lipoprotein niedriger Dichte enthält.

**Revendications**

1. Composition sans sérum, destinée à favoriser la croissance de cellules animales et qui contient un ou plusieurs composants parmi du polyéthylèneglycol en une concentration de 10 % (p/v) ou moins, du poly(alcool vinylique) et du polypropylèneglycol, ladite composition contenant une lipoprotéine légère.

2. Composition selon la revendication 1, qui contient du polyéthylèneglycol.

3. Composition selon la revendication 1, qui contient du polyéthylèneglycol et/ou du poly(alcool vinylique) et une lipoprotéine légère.

4. Composition selon l'une quelconque des revendications 1 à 3, qui est une composition solide.

5. Composition selon l'une quelconque des revendications 1 à 3, qui est une solution.

6. Procédé pour faire croître des cellules animales, comprenant le fait de faire croître des cellules animales dans un milieu sans sérum et qui contient un ou plusieurs composants parmi du polyéthylèneglycol en une concentration de 10 % (p/v) ou moins, du poly(alcool vinylique) et du polypropylèneglycol, ledit milieu contenant une lipoprotéine légère.

7. Procédé selon la revendication 6, dans lequel le milieu utilisé pour faire croître les cellules animales contient du polyéthylèneglycol.

8. Procédé selon la revendication 6, dans lequel le milieu utilisé pour faire croître les cellules animales contient du polyéthylèneglycol et/ou du poly(alcool vinylique) et une lipoprotéine légère.

9. Procédé de production d'une substance biologiquement active, comprenant le fait de faire croître des cellules animales, produisant une substance biologiquement active, dans un milieu sans sérum et qui contient un ou plusieurs composants parmi du polyéthylèneglycol en une concentration de 10 % (p/v) ou moins, du poly(alcool vinylique) et du polypropylèneglycol, et en plus, si on le souhaite, une lipoprotéine légère, la production et l'accumulation de ladite substance dans la culture, et le fait de recueillir cette substance, procédé dans lequel ledit milieu contient une lipoprotéine légère.

FIG. 1

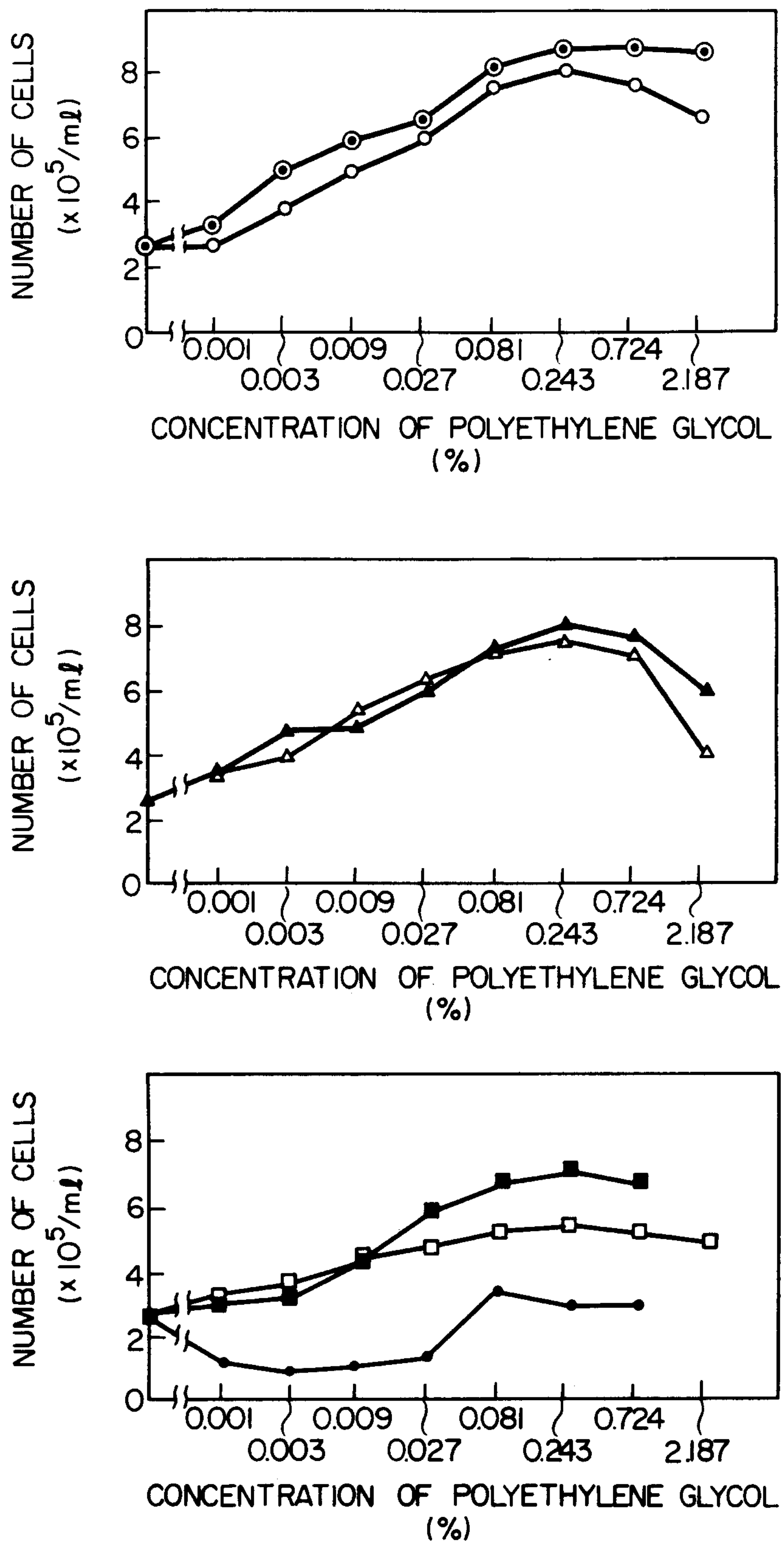
NUMBER OF CELLS
(x 10$^5$/mℓ)
0
2
4
6
8
0.001
0.003
0.009
0.027
0.081
0.243
0.724
2.187
CONCENTRATION OF POLYETHYLENE GLYCOL
(%)
NUMBER OF CELLS
(x10$^5$/mℓ)
0
2
4
6
8
0.001
0.003
0.009
0.027
0.081
0.243
0.724
2.187
CONCENTRATION OF POLYETHYLENE GLYCOL
(%)
NUMBER OF CELLS
(x 10$^5$/mℓ)
0
2
4
6
8
0.001
0.003
0.009
0.027
0.081
0.243
0.724
2.187
CONCENTRATION OF POLYETHYLENE GLYCOL
(%)

FIG. 2

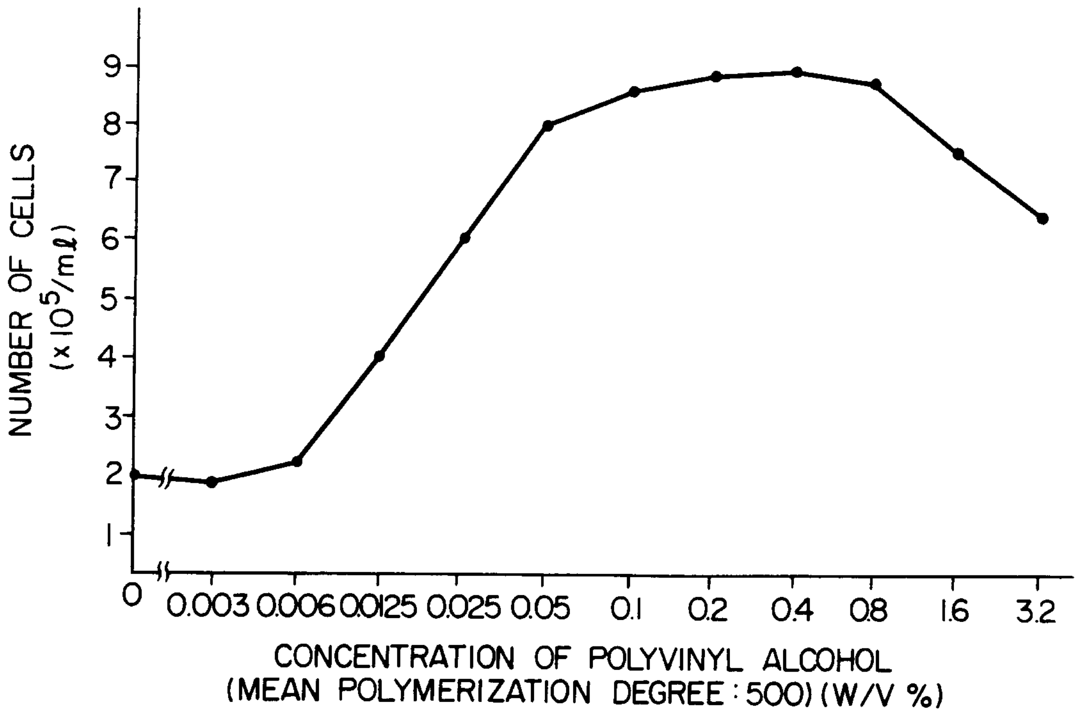
NUMBER OF CELLS
(x10^5/mℓ)
0
1
2
3
4
5
6
7
8
9
0.003 0.006 0.0125 0.025 0.05 0.1 0.2 0.4 0.8 1.6 3.2
CONCENTRATION OF POLYVINYL ALCOHOL
(MEAN POLYMERIZATION DEGREE : 500) (W/V %)

FIG. 3

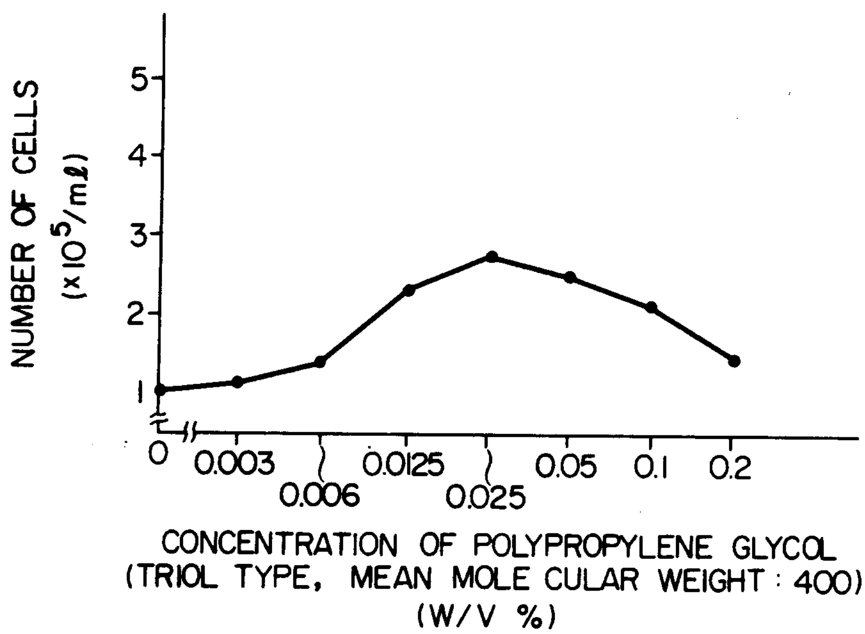
NUMBER OF CELLS
(x10^5/mℓ)
0
1
2
3
4
5
0.003 0.006 0.0125 0.025 0.05 0.1 0.2
CONCENTRATION OF POLYPROPYLENE GLYCOL
(TRIOL TYPE, MEAN MOLE CULAR WEIGHT : 400)
(W/V %)

FIG. 4

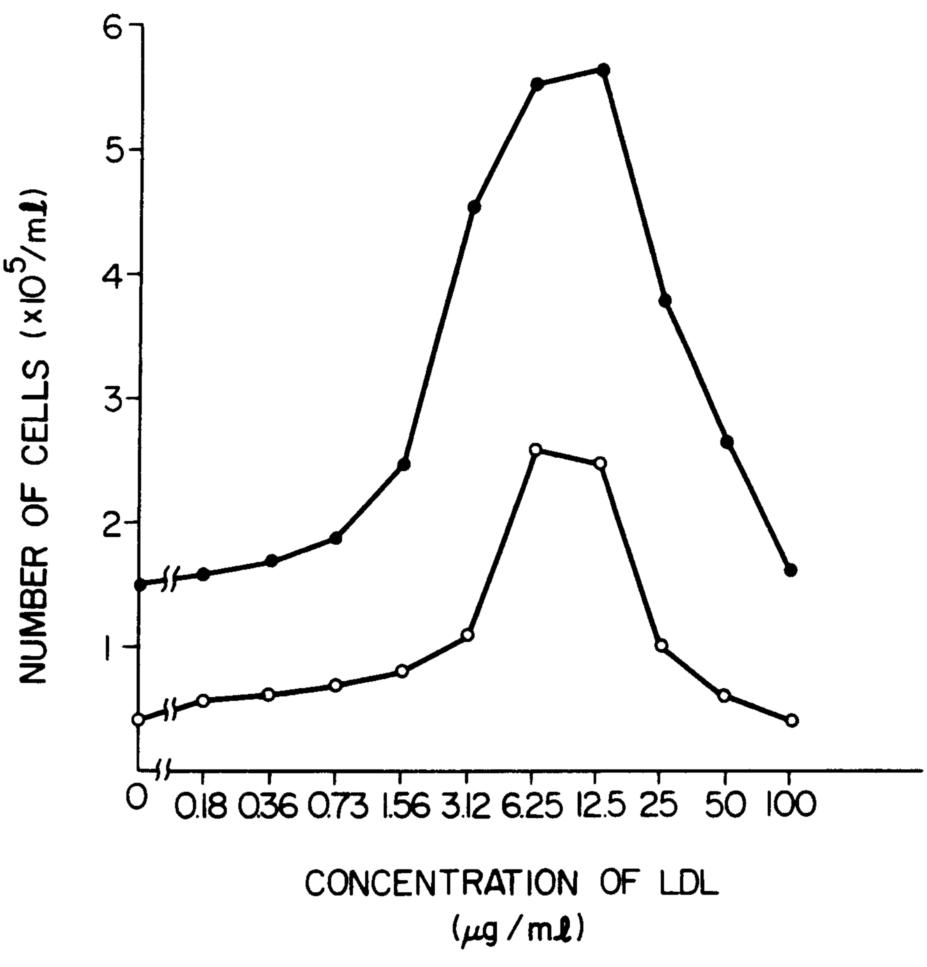
NUMBER OF CELLS (×10$^5$/mℓ)
6
5
4
3
2
1
0
0.18
0.36
0.73
1.56
3.12
6.25
12.5
25
50
100
CONCENTRATION OF LDL
(μg/mℓ)

FIG. 5

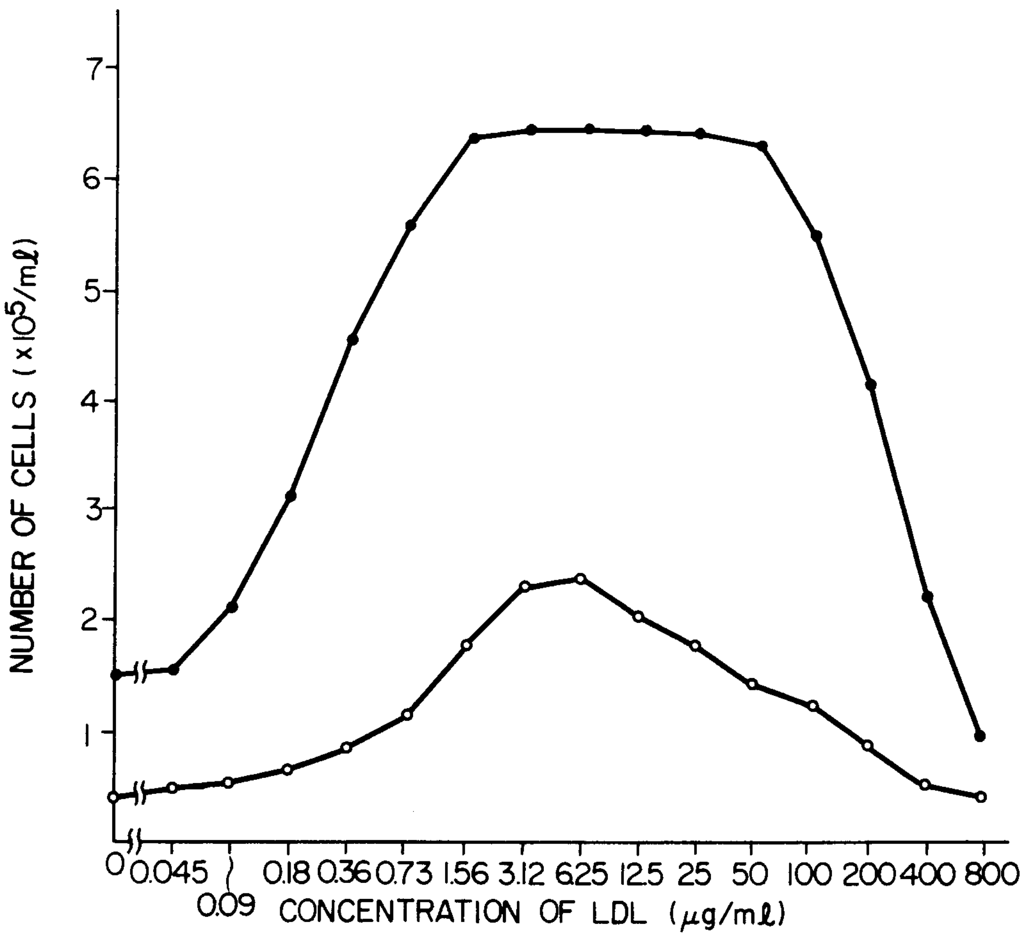
7
6
5
4
3
2
1
NUMBER OF CELLS (x10^5/mℓ)
0
0.045
0.09
0.18
0.36
0.73
1.56
3.12
6.25
12.5
25
50
100
200
400
800
CONCENTRATION OF LDL (μg/mℓ)

FIG. 6

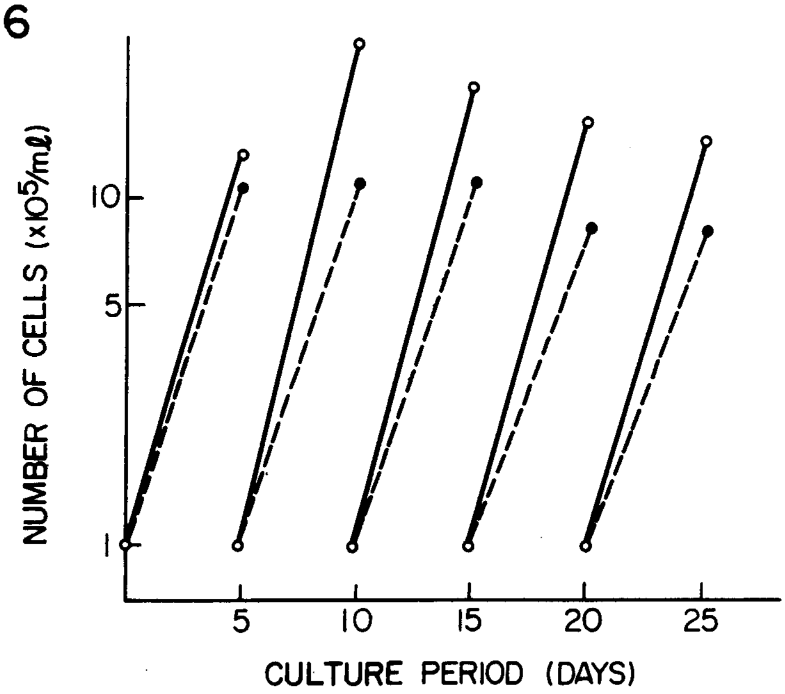
10
5
1
NUMBER OF CELLS (x10^5/mℓ)
5
10
15
20
25
CULTURE PERIOD (DAYS)